# EUROPEAN PATENT APPLICATION

(11) **EP 4 530 023 A1**
(43) Date of publication of application: **02.04.2025**
(21) Application number: 23830258.2
(22) Date of filing: 27.06.2023
(51) Int. Cl.: B25J 3/00, B25J 13/02, A61B 34/35

(54) **CONSOLE AND ROBOT**

(30) Priority: 29.06.2022 CN 202221672160 U
(71) Applicant: Yinuoda Medical Technology (Chengdu) Co., Ltd, Chengdu, Sichuan 610000 (CN)
(72) Inventor: HU, Runchen, Hangzhou, Zhejiang 310051 (CN); SUN, Sinan, Hangzhou, Zhejiang 310051 (CN); XU, Hong, Hangzhou, Zhejiang 310051 (CN); WAN, Yonghong, Hangzhou, Zhejiang 310051 (CN)
(74) Representative: Chung, Hoi Kan
(86) International application number: PCT/CN2023/102815
(87) International publication number: WO 2024/002081

(57) **Abstract**

A console and a robot. The console comprises master controller, the master controller comprises static platform, movable platform, handle and connecting arm. The movable platform is arranged on one side of the static platform; the handle connected to the movable platform; both ends of the connecting arm are respectively hinged and connected to the static platform and the movable platform, the connecting arm includes at least the first support member, the second support member, and the third support member, the first support member to the Nth support member are respectively hinged at the first static hinge point to the Nth static hinge point to the static platform. Among them, the master controller also includes N first drive assemblies for driving each support member to rotate relative to the static platform; the included angle between the static platform and the horizontal plane is less than 45°. The requirements for a rotary drive assembly are reduced, the size of the rotary drive assembly can be reduced while the gravity is balanced to a maximum extent, and the degree of freedom of design of the master controller is improved.

## Description

### TECHNICAL FIELD

The present disclosure relates to the technical field of master-slave control robots, specifically involving a console including a master controller and a robot including the console.

### BACKGROUND

Master-slave control robots typically include a master operating and a slave end. In a master-slave control robot system, the master controller at the master operating serves as an interactive device between the operator and the robot, transmitting information such as position and speeds given by the operator to the slave end. At the same time, it can also transfer environmental information such as forces/torques received by the slave end to the operator, allowing the operator to directly perceive the force information received by the slave end, giving it an operational telepresence and enabling timely and effective control and intervention of the motion of the slave end system. Due to the above-mentioned characteristics of master-slave control robots, their advantages when used as surgical robots in the medical service field have become increasingly prominent.

In surgical robots, the master controller is the carrier of information transfer between the doctor and the slave end manipulator, used to assist the doctor in performing precise operations. The master controller includes a static platform, a movable platform, and multiple connecting arms connected between the static and movable platforms. The handle is installed on the movable platform, and the operator controls the extension and rotation of the connecting arms relative to the static platform through the handle.

The gravity of the master controller can affect the operator's operation experience, if the gravity is not compensated or the gravity balance effect is not ideal, the operator needs to overcome the influence of the master controller gravity during the operation process, in the case of long-term operation, the muscles will feel fatigued. On the other hand, the gravity of the master controller will also affect the operator's perception of the authenticity of force feedback, if the gravity is not compensated, what the operator feels will be the resultant force of the master controller gravity and the feedback force, reducing the immersion of the operation. In addition, the structural design of the master controller's own body will also affect the operator's operation experience. For example, if the structural design of the master controller's is unreasonable, it will increase the fatigue of the operation and reduce the comfort of the operation.

The master controller in the existing technology has the problem of limiting the operating experience of the operator.

### SUMMARY

One of the main inventive purposes of the present disclosure lies in providing a console and a robot to solve the problem of the poor operation experience of the master controller of the console in related technologies.

According to a part of the present disclosure, a console, the console comprising a master controller, the master controller comprises static platform, movable platform, handle and connecting arm. The movable platform is arranged on one side of the static platform. The handle, the handle connected to the movable platform. The connecting arm, both ends of the connecting arm are respectively hinged and connected to the static platform and the movable platform, the connecting arm includes N support members, where N is greater than or equal to 3, the connecting arm comprises at least the first support member, the second support member, and the third support member, the first support member to the Nth support member are respectively hinged at the first static hinge point to the Nth static hinge point to the static platform.

Among them, the master controller also includes N first drive assemblies for driving each support member to rotate relative to the static platform. Among them, the included angle between the static platform and the horizontal plane is less than 45°.

The included angle between the static platform and the horizontal plane is less than or equal to 10°. The included angle is equal to 0° or 5°.

The circumference of the static platform and/or the movable platform is divided into the opposite first side and second side in the circumferential direction, relative to the second side in the circumferential direction, the N support member are arranged around the static platform and/or the movable platform on the first side in the circumferential direction, and the angular range of the second side in the circumferential direction is greater than or equal to 150° and less than 300°.

The angular range of the second side in the circumferential direction can be 180°.

The N support members are evenly arranged in the circumferential direction on the first side in the circumferential direction; and/or the handle is located at the middle position of the second side in the circumferential direction.

The N first drive assemblies are arranged in the area on the first side in the circumferential direction; and/or the first drive assembly that drives the first support member is arranged within the projection area of the static platform.

The first support member, the second support member, and the third support member are respectively connected to the static platform through the first static hinge point, the second static hinge point, and the third static hinge point, the first static hinge point, the second static hinge point, and the third static hinge point are located on the first circle.

The first drive assembly corresponding to the first support member and the first drive assembly corresponding to the third support member are arranged on the same diameter of the first circle, and the output shaft of the first drive assembly corresponding to the first support member and the output shaft of the first drive assembly corresponding to the third support member face the same side.

The first static hinge point to the Nth static hinge point are respectively connected to the virtual center of the static platform to form the first virtual straight line to the Nth virtual straight line. Among them, an included angle is formed between two adjacent virtual straight lines, and the first virtual straight line to the Nth virtual straight line form N included angles. Among them, the included angle formed between the Nth virtual straight line and the first virtual straight line is greater than or equal to 150° and less than 300°.

The first support member to the Nth support member are respectively hinged to the movable platform at the first movable hinge point to the Nth movable hinge point. The movable platform is arranged parallel to the static platform. Among them, the projections of the first support member to the Nth support member from the movable platform to the static platform are respectively coincident with the first virtual straight line to the Nth virtual straight line.

The Nth included angle is equal to 180°; and/or the sum of the N included angles is equal to 360°, and the other included angles except the Nth included angle are equal to each other.

The handle is located between the first support member and the Nth support member; and/or the N is an odd number, the projection of the handle and the (N + 1) / 2-th support member on the static platform is located on the same straight line.

The handle comprises a grip portion, the grip portion is arranged at a predetermined angle with the horizontal plane, and/or the grip portion is arranged at the predetermined angle with the static platform.

The predetermined angle is from 45° to 90°; and/or the included angle between the predetermined angle and the support member and the static platform is close to or equal.

The handle is arranged between the static platform and the movable platform; and/or the movable platform is arranged above the static platform, and the first drive assembly are arranged at the lower end of the corresponding support member.

The driving force of the first drive assembly is transmitted to the corresponding support member through the gear set. The gear set comprises a driving gear connected to the output shaft of the first drive assembly, and a driven gear rotatably arranged on the static platform and meshing with the driving gear, the rotating shaft of the driven gear is connected to the corresponding support member.

The driving gear comprises a first driving gear and a second driving gear which are coaxially and side by side arranged and have the same structure, and the driving teeth of the first driving gear and the driving teeth of the second driving gear are arranged out of phase in the rotation direction; and/or the driven gear comprises a first driven gear and a second driven gear which are coaxially and side by side arranged and have the same structure, the driving teeth of the first driven gear and the driving teeth of the second driven gear are arranged out of phase in the rotational direction.

The offset distance between the driving teeth of the first driving gear and the driving teeth of the second driving gear is approximately equal to the transmission clearance between the first driving gear and the driven gear; and/or the offset distance between the driving teeth of the first driven gear and the driving teeth of the second driven gear is approximately equal to the transmission clearance between the driving gear and the first driven gear.

The driven gear is a sector gear, the sector angle of the sector gear is from 60° to 150°, and the sector gear is set to hollowed-out shapes; and/or the transmission ratio between the driving gear and the driven gear is from 2:1 to 10:1.

Each of the first support member, the second support member, and the third support member comprises a connecting bracket hinged to the static platform, a drive rod moving along the connecting bracket, and a second drive assembly arranged on the connecting bracket, the second drive assembly comprises a mover and a stator, and the mover is fixedly connected to the drive rod and moves synchronously; and/or each support member is rotationally connected to the movable platform through a hinge pair.

The hinge pair comprises first connecting block and second connecting block, the first connecting block is U-shaped, comprises the first support portion and the second support portion opposite to each other, and the connecting support portion connecting one end of the first support portion and the second support portion; and the second connecting block is rotatably mounted on the first support portion and the second support portion. Among them, the connecting support portion is rotatably connected to the corresponding support member through the first rotating shaft, and the second connecting block is rotatably connected to the movable platform through the second rotating shaft. Among them, the central axis of the first rotating shaft is parallel to the extension direction of the first support portion and the second support portion, and is perpendicular to the rotating shaft of the second connecting block rotating on the first support portion and the second support portion.

The console comprising two master controllers, the two master controllers are symmetrically arranged, and the openings of the Nth included angle on both master controllers are oriented towards the same side. Among them, the projection of the handle on the static platform is located within the Nth included angle.

The console comprising two master controllers, and the two master controllers are symmetrically arranged on the bottom plate of the console. Among them, the four first drive assembly that respectively drive the first support member and the third support member of the two master controllers to rotate around their respective static hinge points are arranged along the same straight line.

The first drive assembly that respectively drives the second support member of the two master controllers to rotate around their respective static hinge points, is arranged in parallel; and/or the two first drive assembly that respectively drive the first support member of the two master controllers are located beneath the static platform.

According to another aspect of the present disclosure, a robot is provided, which may include the console as described above.

The robot can be a surgical robot.

According to the embodiments of the present disclosure, by setting the included angle between the static platform and the horizontal plane to be less than 45°, the gravity can be balanced to a large extent, while reducing the requirements for the rotational drive assembly, reducing the volume of the rotational drive assembly, and increasing the design space of the master controller of the console.

According to the embodiments of the present disclosure, the transmission gap is eliminated by the staggered arrangement of the two gears, avoiding the jitter phenomenon caused by delayed meshing.

According to the embodiments of the present disclosure, by setting the sector gear and the appropriate transmission ratio, the rotational inertia is reduced, the lagging sensation is decreased, and the operator's hand feel is improved.

According to the embodiments of the present disclosure, by designing the hinge pair including the U-shaped connecting member, the interference between the movable platform and the hinge pair is avoided, expanding the motion range of the movable platform.

### BRIEF DESCRIPTION OF DRAWINGS

The above and/or other purposes and advantages of the present disclosure will become clearer through the description of embodiments in conjunction with the accompanying drawings, among which:
FIG. 1 shows a structure diagram of the console;
FIG. 2 shows a structure diagram of the master controller;
FIG. 3 shows a structure diagram of the other state of the master controller in FIG. 2;
FIG. 4 shows a schematic diagram of the distribution of the gravitational component of a support member;
FIG. 5 shows a planar schematic diagram of the static platform and presents a schematic diagram of the arrangement of the three static hinge points relative to the center of the static platform;
FIG. 6 shows a schematic structural diagram of the first embodiment of the gear set according to the embodiment of the present disclosure;
FIG. 7 shows a schematic structural diagram of the second embodiment of the gear set according to the embodiment of the present disclosure;
FIG. 8 shows the structural plan view of the gear set according to the second embodiment;
FIG. 9 shows an enlarged diagram of Part A of FIG. 7;
FIG. 10 shows a schematic structural diagram of the hinge pair according to the embodiment of the present disclosure;
FIG. 11 shows a three-dimensional view of the first connecting block of the hinge pair according to the present disclosure;
FIG. 12 shows a three-dimensional view of the second connecting block of the hinge pair according to the present disclosure.

### Explanation of reference signs:

10 - console; 11 - bottom plate; 20 - master controller; 100 - static platform; 100' - support platform; 110 - first static hinge point; 120 - second static hinge point; 130 - third static hinge point; 111 - virtual center; 200 - movable platform; 300 - connecting arm; 310 - first support member; 320 - second support member; 330 - third support member; 340 - connecting bracket; 350 - second drive assembly; 351 - mover; 352 - stator; 360 - drive rod; 400 - first drive assembly; 500 - gear set; 510 - driving gear; 520 - driven gear; 511 - first driving gear; 512 - second driving gear; 521 - first driven gear; 522 - second driven gear; 525 - rotating shaft; 600 - handle; 610 - grip portion; 700 - first connecting block; 800 - second connecting block; 710 - first support portion; 720 - second support portion; 730 - connecting support portion; 731 - first shaft hole; 711, 721 - hole; 810 - main body; 820 - convex shaft; 831 - second shaft hole.

### DETAILED DESCRIPTION OF THE EMBODIMENTS

Now the exemplary embodiments are described more comprehensively by referring to the accompanying drawings. However, it should not be understood that the embodiments disclosed in the present disclosure are limited to the embodiments described herein. The same reference signs in the figure represent the same or similar structures, therefore their detailed descriptions will be omitted.

When describing the hinge pairs, master controller s and console in accordance with the embodiments of the present disclosure, the surgical robot will be introduced as an example. However, those skilled in the art will realize that the master controller in accordance with the present disclosure is not limited to being applied to surgical robots, but can be a master-slave control robot used in various other fields.

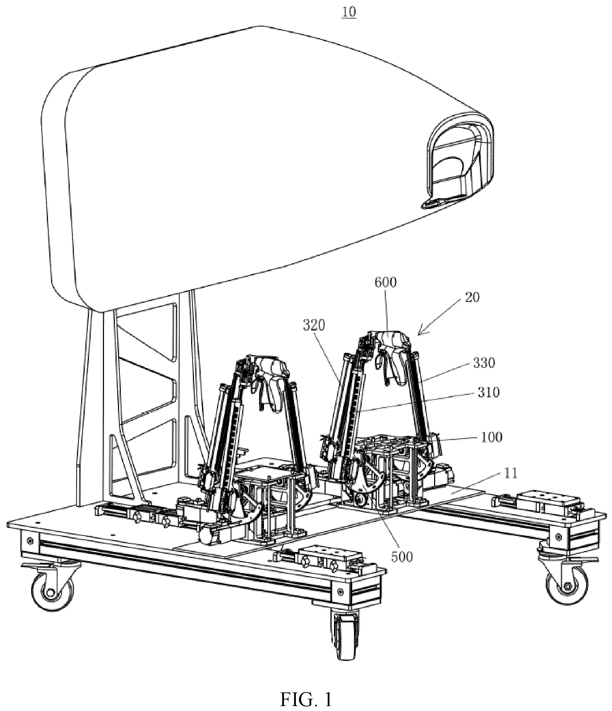

FIG. 1 shows a structural schematic diagram of the master controller applied to the console of the surgical robot according to the embodiment of the present disclosure.

Surgical robots typically comprise a console 10, a slave end (not shown), and imaging equipment (not shown). As shown in FIG. 1, the console 10 may comprise a master controller 20, the slave end may be a mechanical arm system that performs the surgery, and the imaging equipment may include a display for showing images of the patient's lesion and other relevant information. The master controller 20 is set up on the doctor's console, and the operator (doctor) can control the operation of the slave end by operating the master controller 20. The force exerted on the instrument at the end of the slave end can be fed back to the master controller 20 through a force sensor to establish a mechanical model between the console 10 and the slave end.

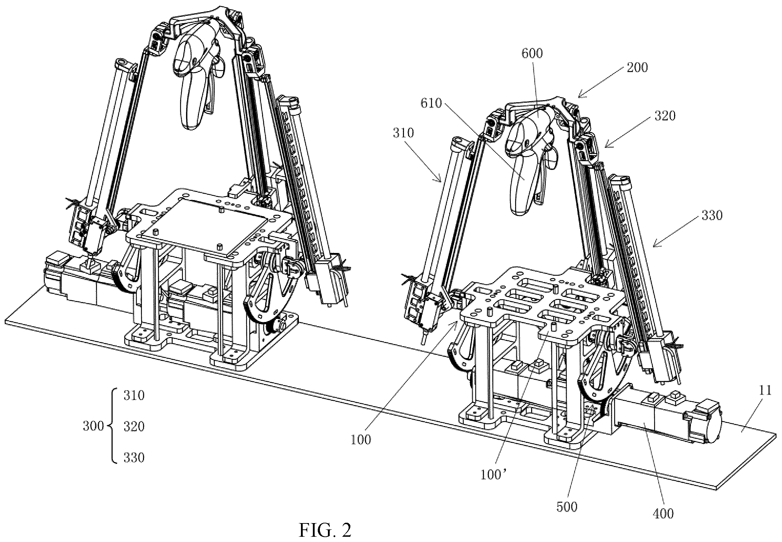

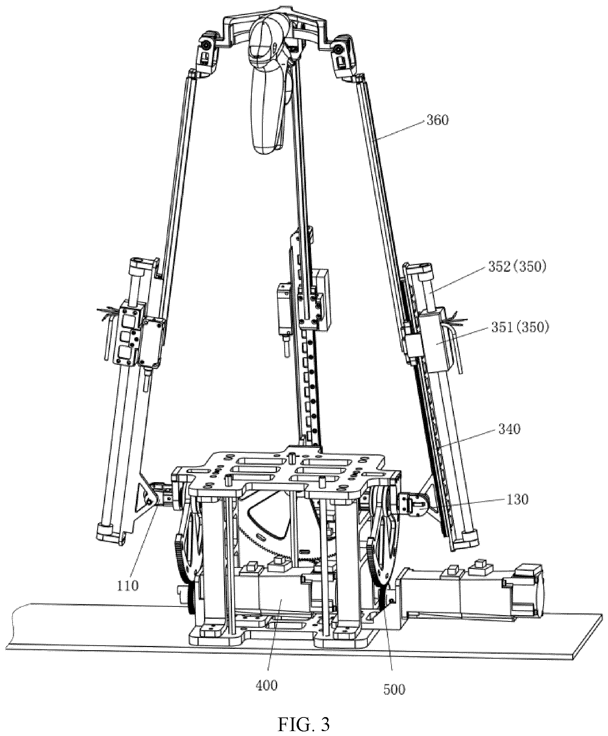

FIGs. 2 and 3 show the stereograms of the master controller in different operating states according to the embodiments of the present disclosure. Among them, FIG. 2 shows the stereogram of the master controller in the initial state. In the following description, unless otherwise explicitly stated, the structural positional relationship when the master controller 20 is in the initial state is introduced.

As shown in FIGs. 2 and 3, the master controller 20 according to the embodiments of the present disclosure may include a static platform 100, a movable platform 200, and a connecting arm 300. The movable platform 200 may be arranged on one side of the static platform 100, and both ends of the connecting arm 300 may be hinged and connected to the static platform 100 and the movable platform 200 respectively. Among them, the connecting arm 300 may include N support members, where N is a natural number greater than or equal to 3. the connecting arm 300 may include at least a first support member 310, a second support member 320, and a third support member 330. The embodiments of the present disclosure show the situation where the connecting arm 300 includes three support members in the accompanying figures, but the present disclosure is not limited to this.

In the existing technology, static platform set perpendicular to the ground in general, the gravity of the master controller itself needs to be balanced by a rotating motor mounted on the static platform, increased the rotaring motor load. Therefore, the rotating motor requires a larger power, and the larger the power of the rotating motor, the larger the volume, resulting in the need for a larger space to install the rotating motor. If the volume of the rotating motor is large, the connecting arm is prone to interference with the rotating motor, resulting in the limited motion range of the master controller. Therefore, it is difficult to simultaneously consider the gravity balance effect and the motion range, limiting the development of the master controller.

### To solve the above-mentioned problems:

According to the embodiments of the present disclosure, the included angle between the static platform 100 and the horizontal plane can be less than 45°. Preferably, the included angle between the static platform 100 and the horizontal plane can be less than or equal to 35°, 30°, 25°, 15°, or 10°, etc. Preferably, the included angle between the static platform 100 and the horizontal plane can be less than or equal to 5°. More preferably, the included angle between the static platform 100 and the horizontal plane can also be equal to 0°, that is, the static platform 100 is approximately parallel to the horizontal plane.

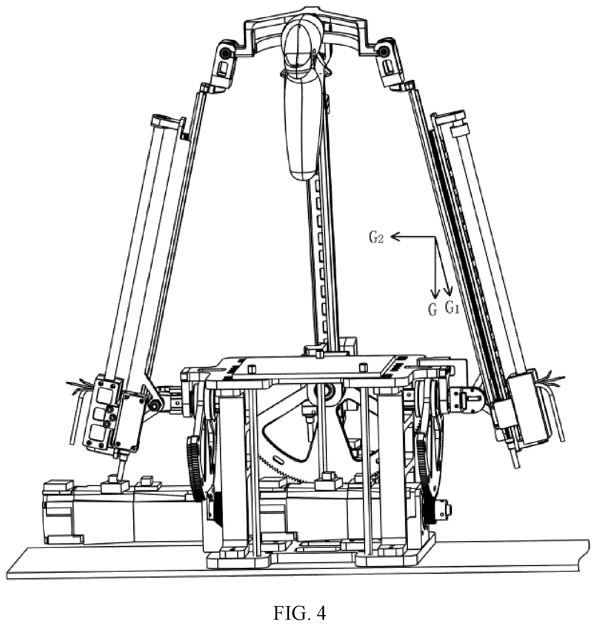

FIG. 4 shows the distribution of the gravitational component of a support member. As shown in the figure, the gravity G of each support member can be decomposed into a first component G1 along its length direction and a second component G2 approximately parallel to the static platform and towards the corresponding static hinge point. When the included angle between the static platform 100 and the horizontal plane is less than 45°, compared to the situation where the static platform 100 is arranged perpendicularly to the horizontal plane, more of the gravitational component (i.e., the first component G1) is distributed along the length direction of each support member. Therefore, when balancing the gravity of the support member, most of the gravity will be compensated by the linear drive assembly (also known as the second drive assembly ) that drives each support member to move in a linear direction, while the rotational drive assembly (also known as the first drive assembly ) used to rotate each support member can provide a smaller torque, thereby reducing the requirements for the output torque of the rotational drive assembly and allowing the rotational drive assembly to retain a larger margin. Secondly, it also enables a more balanced load distribution between the linear drive assembly and the rotational drive assembly during the gravity balancing process. Especially in the initial state of the master controller , when each drive assembly is locked, the balance of each load is better. As the included angle between the static platform 100 and the horizontal plane becomes smaller, the value of the first component G1 of the gravity G becomes larger, so the more gravity needs to be compensated by the linear drive assembly , and the lower the requirements for the rotational drive motor, thereby achieving a better gravity balancing effect of the master controller. **In** one implementation, due to the larger margin of the rotational drive assembly , the load distribution between the drive assembly s is more balanced, making it easier to achieve real-time force feedback, allowing the operator to feel the force feedback at the driven end more realistically.

Due to the adjustment of the position of the static platform of the master controller, the control angle between the operator's hand and the master controller has been changed. As a result, the operator has a better hand feel and is less prone to fatigue. Its design complies with ergonomics. Additionally, due to the reduced requirements for the rotational drive assembly, in one implementation, the volume of the rotational drive assembly can be reduced, thereby expanding the design range of the available space and enabling the structure of the master controller. It is more compact and can expand the movement range of the master controller at the same time.

Furthermore, the movable platform 200 can be arranged above the static platform 100 in the vertical direction, and the movement of each support member is driven through the first drive assembly and the second drive assembly, enabling the movable platform 200 to move relative to the static platform 100. According to the embodiments of the present disclosure, the movable platform 200 is arranged parallel to the static platform 100, and the movable platform can have 6 degrees of freedom.

However, the present disclosure is not limited to this. According to the layout requirements of the space, the movable platform 200 can also be arranged beneath the static platform 100.

It should be noted that the "static platform" typically refers to the plane on which the static hinge points of each support member connected by the connecting arm 300 are located. In one implementation, the "static platform" refers to a virtual plane; in another implementation, the "static platform" can also be a physical platform. In this implementation, the static platform 100 is parallel to the support platform 100' that supports each support member at the static hinge points. Therefore, the included angle relationship between the static platform 100 and the horizontal plane is the same as the relationship between the actual support platform 100' and the horizontal plane. In another implementation, the static platform 100 can also be coplanar with the actual support platform 100'. For ease of understanding and description, the static platform 100 can be understood as the actual support platform 100' in the following text. Both the static platform 100 and the actual support platform 100' will be described as "static platform 100" in the following text.

The concept of "movable platform" is similar to that of "static platform". Specifically, "movable platform" usually refers to the plane where the movable hinge point of each support member connected by the connecting arm 300 are located. In one implementation, "movable platform" refers to a virtual plane; in another implementation, "movable platform" can also be a physical platform. In other implementations, the physical movable platform can be parallel to the planes where the movable hinge point are located. In this implementation, the physical movable platform and the planes where the movable hinge point are located are coplanar. Therefore, in the following text, "movable platform 200" can be understood as either a virtual platform or an actual platform. However, for the sake of ease of understanding, the physical movable platform 200 is described in the following text.

In this manual, "gravity compensation" refers to the output torque of the drive assembly used to balance the gravity. "Gravity balance" refers to the output torque of the drive assembly being able to counteract the gravity.

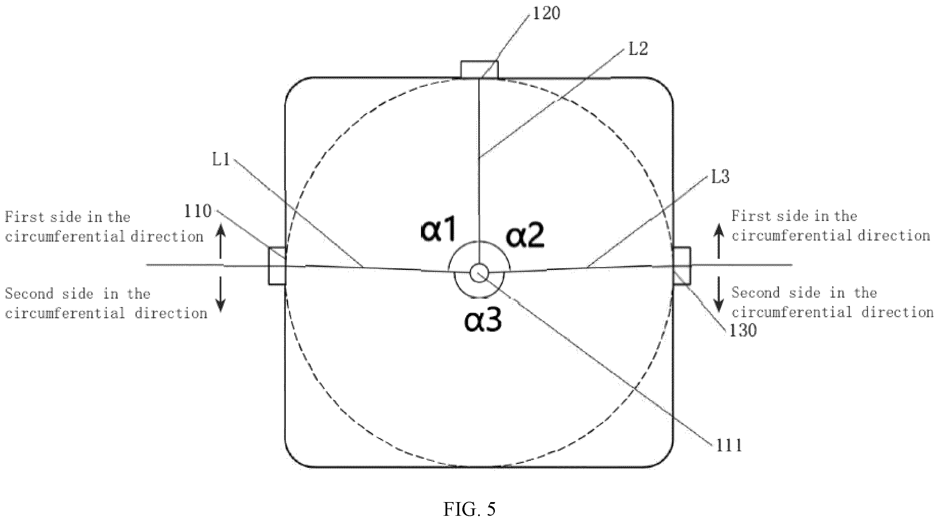

FIG. 5 shows a schematic planar diagram of the positional relationship between the three static hinge points of the three support members and the static platform in accordance with the embodiment of the present disclosure.

The structural design of the master controller in the current design is prone to interference with the operator. To avoid the movement range of the master controller, the operator usually stays far away from the master controller, which greatly affects the operator's operation experience and increases the fatigue of the operation. Therefore, it is difficult to take into account both the movement range and the operation at the same time. And restrict the development of the master controller.

### To solve the above-mentioned problems:

Combining FIGs. 2 and 3, the first support member 310, second support member 320, and third support member 330 of the connecting arm 300 can be hinged to the static platform 100 at the first static hinge point 110, second static hinge point 120, and third static hinge point 130, respectively. The first virtual straight line L1 connecting the first static hinge point 110 and the virtual center 111 of the static platform 100 forms a first included angle α1 with the second virtual straight line L2 connecting the second static hinge point 120 and the virtual center 111 of the static platform 100, and the second virtual straight line L2 forms a second included angle α2 with the third virtual straight line L3 connecting the third static hinge point 130 and the virtual center 111 of the static platform 100. The first virtual straight line L1 and the third virtual straight line L3 form a third included angle α3, which can be greater than or equal to 150° and less than 300°. In one implementation, the sum of the first included angle α1, second included angle α2, and third included angle α3 is equal to 360°.

In one embodiment, the distribution structure between the movable platform 200 and each support member is the same as that between the static platform 100 and each support member. In this case, the distribution structures of the two can be the same or different on the same master controller. Additionally, the first support member 310, the second support member 320, and the third support member 330 can be hinged to the movable platform 200 at the first moving hinge point, the second moving hinge point, and the third moving hinge point, respectively. The included angles formed by the connections between each moving hinge point and the virtual center of the movable platform 200 and the corresponding static hinge point and the virtual center 111 of the static platform 100 are corresponding to each other. That is to say, in the initial state, the static platform 100 and the movable platform 200 are set parallel to each other, and the connection between the virtual center 111 of the static platform 100 and the virtual center of the movable platform 200 is perpendicular to the static platform 100 and the movable platform 200. Moreover, the projections of the first support member 310, the second support member 320, and the third support member 330 on the static platform 100 coincide with the first virtual straight line L1, the second virtual straight line L2, and the third virtual straight line L3, respectively.

In this manual, "projection on the static platform" refers to the projection of the corresponding component onto the static platform 100 in the direction perpendicular to the static platform 100.

According to the embodiments of the present disclosure, when the operator controls the master controller to operate, the third included angle α3 can be used to accommodate the operator's body (such as legs and hands), avoiding interference between the master controller and the operator. At the same time, it also maintains an appropriate operating space between the operator and the master controller , allowing both the motion range and the comfort of the operation to be taken into account simultaneously.

According to the embodiments of the present disclosure, the master controller also includes a handle 600, which can be arranged on the movable platform 200. The operator holds the handle 600 to operate. When the handle is located between the first support member 310 and the third support member 330, the first support member 310 and the third support member 330 are close to the operator set. If the third Angle α3 between the first virtual straight line and the third virtual straight line is small (e.g., α3<150°), the space used to accommodate the operator's body (e.g., legs, hands) is small, there is a problem that the lower end of the first support member 310 and the third support member 330 are prone to interference with the operator's legs, and the upper end of the first support member 310 and the third support member 330 are prone to interference with the operator's hands.

In this disclosure, the third included angle α3 is set to be greater than or equal to 150° and less than 300°, which can leave a larger activity space for the operator and thereby avoid the above-mentioned problems.

According to the preferred embodiment of the present disclosure, the third included angle α3 can be 180°. Among them, the first included angle α1 and the second included angle α2 can be equal to each other. In other embodiments, the third included angle α3 can be 160°, 200°, 220°, 245°, 260°, or 285°, etc. However, the present disclosure is not limited to this. The first included angle α1 and the second included angle α2 can also be unequal.

However, the attached figure only shows the situation where the connecting arm 300 includes three support members, but the connecting arm 300 according to the present disclosure can also include a greater number of support member.

When the connecting arm 300 includes N (N > 3) support members, the arrangement of each support member is similar to that when there are three support members. Specifically, when the connecting arm 300 includes N (N > 3) support members, the first support member 310 to the Nth support member can be hinged to the static platform 100 at the first static hinge point 110 to the Nth static hinge point respectively. The first static hinge point 110 to the Nth static hinge point are respectively connected to the virtual center 111 of the static platform 100 to form the first virtual straight line L1 to the Nth virtual straight line LN (not shown), where an included angle is formed between adjacent virtual straight lines. Therefore, a total of N included angles are formed between adjacent pairs of the first virtual straight line L1 to the Nth virtual straight line LN. In this case, the Nth included angle formed between the Nth virtual straight line LN and the first virtual straight line L1 is greater than or equal to 150° and less than 300°. Preferably, the sum of the N included angles is equal to 360°. Preferably, the Nth included angle is approximately 180°.

Similarly, the first support member 310 to the Nth support member are respectively hinged to the movable platform 200 at the first moving hinge point to the Nth moving hinge point. The included angles formed by the connection lines from the first moving hinge point to the Nth moving hinge point to the virtual center of the movable platform 200 correspond respectively to the included angles formed by the connection lines from the corresponding static hinge points to the virtual center 111 of the static platform 100. That is, when projected onto the static platform 100, the first support member 310 to the Nth support member respectively coincide with the first virtual straight line L1 to the Nth virtual straight line LN.

Furthermore, the other included angles except the Nth included angle can be the same as each other.

According to the embodiments of the present disclosure, the circumference of the static platform 100 and/or the movable platform 200 can be divided into the opposite first side and second side in the circumferential direction. Relative to the second side in the circumferential direction, the N support members are arranged around the static platform 100 and/or the movable platform 200 on the first side in the circumferential direction of the static platform 100 and the movable platform 200. Preferably, the angular range of the second side in the circumferential direction is greater than 120°. Preferably, the angular range of the second side in the circumferential direction is greater than or equal to 150° and less than 300°. Preferably, the angular range of the second side in the circumferential direction is 180°. In one implementation mode, the N support members are evenly arranged in the circumferential direction on the first side in the circumferential direction to improve the balance of gravity distribution. In other embodiments, the angular range of the second side in the circumferential direction can be 160°, 200°, 220°, 245°, 260°, or 285°, etc. This is used to maintain an appropriate operating space between the operator and the master controller, taking into account both the motion range and the operating comfort.

The master controller also include N first drive assemblies 400 for driving each support member to rotate around its respective static hinge point relative to the static platform 100. The N first drive assemblies 400 are arranged in the area on the first side in the circumferential direction. The specific arrangement of the first drive assembly 400 will be described hereinafter.

According to the embodiments of the present disclosure, the handle 600 can be arranged on the movable platform 200 and can be located between the first support member 310 and the Nth support member, or the handle 600 can be located on the second side in the circumferential direction. The handle 600 is installed on the other side opposite to the installation position of each support member, which is conducive to expanding the operator's operating space and avoiding the possibility of interference. Preferably, the handle 600 is located at the middle position of the second side in the circumferential direction. The handle is set at the middle position, which is conducive to evenly distributing the weight of the handle to each support member, making the load of each drive assembly more balanced, so as to improve the operator's operating hand feel.

Furthermore, the handle 600 may include a grip portion 610, which is arranged at an angle to the horizontal plane and/or to the static platform 100, to facilitate the operator to operate the master controller by grasping the grip portion 610, thereby enhancing the operating experience of the master controller. Preferably, the angle is between 45 and 90°. As a further preference, the angle is between 75 and 90°. In one implementation mode, the angle is 50°, 60°, 65°, 70°, 80°, or 85°. In another implementation mode, the angle is close to or equal to the included angle between the support member and the static platform 100 in the initial state. In such cases, the comfort of the operation can be further improved.

According to the embodiments of the present disclosure, the handle 600 can be arranged vertically between the static platform 100 and the movable platform 200. This can reduce the volume of the master controller within the entire console and further expand the motion range of the main operation. Additionally, when the number N of the support member is an odd number, the projection of the handle 600 and the (N + 1) / 2-th support member on the static platform 100 can be located on the same straight line. This is conducive to improving the experience of the operator and making the load of each drive assembly more balanced.

When the master controller according to the embodiment of the present disclosure is installed on the console, the master controller is installed on the bottom plate 11 of the console. Among them, the opening of the Nth included angle or the second side in the circumferential direction face towards the outside of the console, that is, towards the operator. Here, the bottom plate 11 is approximately parallel to the horizontal plane, that is, the ground.

In another implementation mode, two master controllers can be symmetrically arranged on the console, such that the openings of the Nth clamping angles of the two master controllers face the same side. For example, the openings of the Nth clamping angles of the two master controllers face the position where the operator is located, thereby providing a larger activity space for the operator between the first support member and the Nth support member, so that the support member do not interfere with the operator's legs. In this case, the two handles 600 are also located on the same side, and their projections on the static platform are within the Nth clamping angle, allowing the operator to conveniently hold the grip 610 of the handle 600.

In the following description, the connecting arm 300 is described as including the first support member 310, the second support member 320, and the third support member 330. When the connecting arm 300 includes a different number of support member, similar adjustments or settings can be made, so the relevant descriptions will be omitted.

According to the embodiments of the present disclosure, as shown in FIGs. 2 to 4, each of the first support member 310, the second support member 320 and the third support member 330 has a linear shape and includes a first end (upper end) and a second end (lower end) opposite to the first end. The first end is hinged to the movable platform 200, and the second end is respectively adjacent to the first static hinge point 110, the second static hinge point 120 and the third static hinge point 130. That is, the distance between the corresponding static hinge point and the second end is approximately one-tenth of the length of the support member or less. Thus, when the operator operates the master controller , the interference between the lower end of the support member and other structures can be effectively avoided, providing a larger space for the arrangement of the rotational drive assembly (i.e., the first drive assembly 400), which will be described hereinafter.

As mentioned above, the master controller may also include three first drive assemblies (i.e., rotational drive assembly) 400 that respectively drive the first support member 310, the second support member 320, and the third support member 330 to rotate around their respective static hinge points.

The volume of the first drive assembly 400 is usually large. Since the included angle between the static platform and the horizontal plane in the embodiments of the present application is less than 45°, the first drive assembly 400 does not need to provide much torque to balance the gravity of the corresponding support member. Therefore, the volume of the first drive assembly 400 can be appropriately reduced. At the same time, since the lower ends of the first support member 310, the second support member 320, and the third support member 330 are adjacent to the static platform 100, there is a large space at the lower ends of the first support member 310, the second support member 320, and the third support member 330 for arranging the first drive assembly 400, thereby expanding the space for arranging the first drive assembly 400. According to the embodiments of the present disclosure, the first drive assembly 400 can be arranged below the corresponding support member.

As shown in FIGs. 1 and 2, the first drive assembly 400 that drives the first support member 310 can be arranged beneath the static platform 100. That is, the first drive assembly 400 that drives the first support member 310 can be arranged within the projection area of the static platform 100, leaving a large space on one side of the master controller for operation. The side where the operator is located. Additionally, the attached figure shows the situation where the first included angle α1 and the second included angle α2 are respectively 90°, and the third included angle α3 is 180° to arrange the first support member 310, the second support member 320 and the third support member 330. In this case, the first drive assembly 400 that drives the first support member 310 and the third support member 330 respectively can be arranged on the same diameter of the virtual circle (hereinafter referred to as the first circle) formed by the first static hinge point 110, the second static hinge point 120 and the third static hinge point 130, and the first drive assembly 400 that drives the second support member 320 can be arranged perpendicularly to this same diameter.

When the two master controllers are installed on the bottom plate 11 of the console 10, the two master controllers can be symmetrically arranged. The first support member 310 of the two master controllers can be arranged close to each other relative to the two third support members 330, that is, the two first support members 310 of the two master controllers 20 are adjacent. Therefore, the first drive assembly that drive the two first support members 310 of the two master controllers 20 can be respectively arranged below the static platform 100 to avoid extending outward relative to the static platform 100, so that the distance between the two master controllers 20 is appropriate, conforms to the operation habits of the operator's hands, and leaves room for the operator's movement. In addition, the four first drive assembly 400 that respectively drive the first support member 310 and the third support member 330 of the two master controllers can be arranged on the same straight line. The two first drive assemblies 400 that drive the second support member 320 of the two master controllers can be arranged in parallel with each other.

Furthermore, the output shaft of the first drive assembly 400 that drives the first support member 310 and the output shaft of the first drive assembly 400 that drives the third support member 330 can be oriented towards the same side, and the output shaft of the first drive assembly 400 that drives the second support member 320 can be perpendicular to the output shaft of the first drive assembly 400 that drives the first support member 310 and the second support member 320.

Again referring to FIGs. 1 to 3, according to the embodiments of the present disclosure, each of the first support member 310, the second support member 320 and the third support member 330 comprises a connecting bracket 340 hinged to the static platform 100, a drive rod 360 moving along the connecting bracket 340, and a second drive assembly (i.e., linear drive assembly) 350 arranged on the connecting bracket 340. The second drive assembly 350 may include a mover 351 and a stator 352, and the mover 351 can be fixedly connected to the drive rod 360 and move synchronously. According to the embodiments of the present disclosure, the second drive assembly 350 can be a linear motor, the stator can be a linear shaft arranged along the length direction of the connecting bracket 340, and the mover 351 can be a slider moving along the linear shaft. In this way, when the handle 600 moves, the slider moves on the linear shaft, and the drive rod 360 moves synchronously and in the same direction as the slider.

The first component G1 of the gravity G of each support member along its length direction can be balanced by the second drive assembly 350, and the volume of the linear drive assembly has a relatively small influence on the motion range of the master controller, resulting in a better effect of gravity balance.

According to the embodiments of the present disclosure, the drive rod 360 can be made of carbon fiber, which is very lightweight. During the movement of the movable platform 200, the influence of their weight on the center of gravity of the support member can be ignored, and the balancing process is simpler.

According to the embodiment disclosed in this invention, the driving force of the First drive assembly 400 can be transmitted to the corresponding support member through the gear set 500.

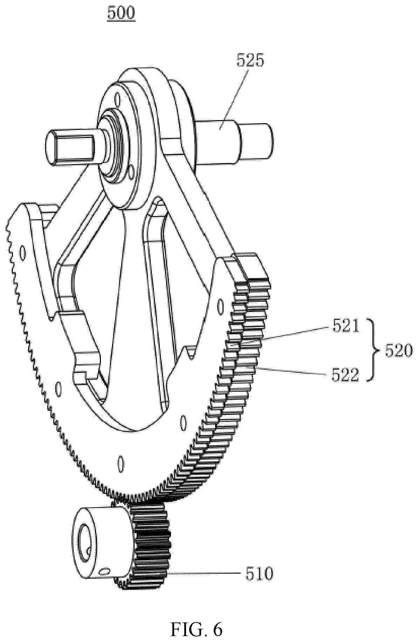

FIG. 6 shows a schematic diagram of the structure of the first embodiment of the gear set according to the embodiment of the present disclosure. As shown in FIG. 6, the gear set 500 may include a driving gear 510 connected to the output shaft of the first drive assembly 400, and a driven gear 520 rotatably arranged on the static platform 100 and meshing with the driving gear 510. The rotating shaft 525 of the driven gear 520 is connected to the corresponding support member.

As shown in FIG. 6, the driving gear 510 may include a gear, and the driven gear 520 may include a first driven gear 521 and a second driven gear 522 that are coaxially and side by side arranged and have the same tooth structure. The driving teeth of the first driven gear 521 and the driving teeth of the second driven gear 522 may be arranged out of phase in the rotation direction. The phase difference between the driving teeth of the first driven gear 521 and the driving teeth of the second driven gear 522 is approximately equal to the transmission gap between the driving gear 510 and a single driven gear. The identical tooth structure mentioned for the first driven gear 521 and the second driven gear 522 refers to the same pitch circle and pitch of the gears, without the requirement that the axial lengths (for example, the thickness of the gears) of the two gears be exactly the same.

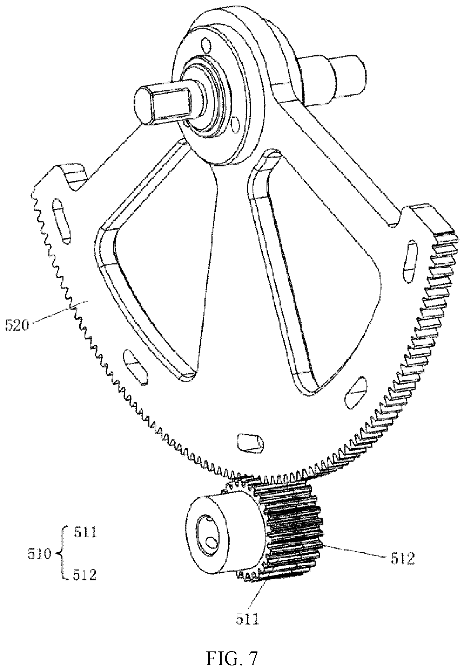

However, the embodiments of the present disclosure are not limited to this. FIG. 7 shows a structural schematic diagram of the second embodiment of the gear set according to the embodiments of the present disclosure. As shown in the figure, the driving gear 510 may include the first driving gear 511 and the second driving gear 512 that are coaxially and side by side arranged and have the same tooth structure. The driving teeth of the first driving gear 511 and the driving teeth of the second driving gear 512 can be arranged out of phase in the rotation direction. Similar to the above embodiment, the out-of-phase distance between the driving teeth of the first driving gear 511 and the driving teeth of the second driving gear 512 is approximately equal to the transmission gap between a single driving gear and the driven gear 520. Similarly, the tooth structure of the first driving gear 511 and the second driving gear 512 mentioned here refers to the same pitch circle and pitch of the gears, and it is not required that the axial lengths of the two gears be exactly the same.

According to the embodiments of the present disclosure, "approximately equal to" indicates that the misaligned distance between the transmission gears is almost equal to or slightly less than the transmission gap between the driving gear 510 and the driven gear 520.

According to the above-mentioned embodiments, the transmission clearance can be almost eliminated. Therefore, regardless of whether the driving gear 510 rotates forward or backward, the driving gear 510 can mesh with the driven gear 520 in real time without delayed meshing, thereby avoiding the phenomenon of jitter.

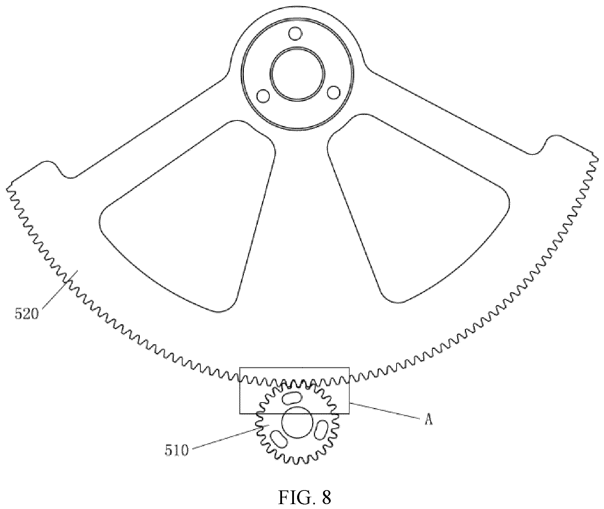

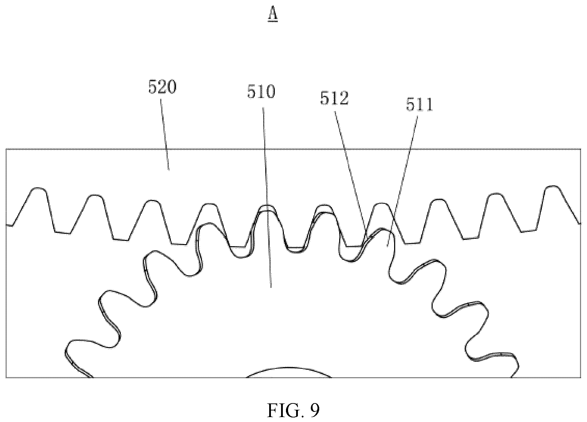

FIGs. 8 and 9 show the schematic diagram of the principle for eliminating the transmission gap in the case of the implementation of FIG. 7. As shown in FIG. 9, due to processing errors, there exists a transmission clearance between each driving gear and the driven gear 520. Taking the direction shown in FIG. 9 as an example, when the right side of the driving tooth of the first driving gear 511 is in contact with the left side of the driving tooth of the driven gear 520, the left side of the driving tooth cannot come into contact with the driving tooth of the driven gear 520 on its left side. This clearance can be understood as a "transmission clearance". In this case, a second driving gear 512 that is coaxial with the first driving gear 511 and has the same tooth shape is provided, so that the driving tooth of the second driving gear 512 is slightly offset from the driving tooth of the first driving gear 511 in the rotation direction. This offset distance can be approximately equal to the transmission clearance, so that the left side of the driving tooth of the second driving gear 512 comes into contact with the right side of the driving tooth of the driven gear 520. This can eliminate the transmission clearance caused by processing errors. The gear set of the implementation shown in FIG. 6 eliminates the transmission clearance based on the same setting principle, so its detailed description is omitted.

According to the embodiments of the present disclosure, the driven gear 520 can be a sector gear, thereby enabling the reduction of the space occupied by the driven gear when a larger transmission ratio is set, reducing the rotational inertia of the overall structure and allowing the operator to feel lighter.

According to the embodiments of the present disclosure, the sectorial angle of the sector gear can be from 60° to 150° to meet the rotational requirements of the corresponding support member. According to one embodiment of the present disclosure, the sectorial angle of the sector gear can be from 100° to 120°. Preferably, the sectorial angle of the sector gear can be 120°.

According to the embodiments of the present disclosure, the transmission ratio of the driving gear 510 and the driven gear 520 can be set from 2:1 to 10:1. Preferably, the transmission ratio can be from 5:1 to 8:1. More preferably, the transmission ratio can be set at 8:1. When this transmission ratio is selected, the output torque at the static hinge point is relatively high.

According to the embodiments of the present disclosure, the sector gear can be set in a hollowed-out shape and can be fabricated from plastic, thereby further reducing the rotational inertia, lowering the lagging sensation, and providing a better hand feel for the operator.

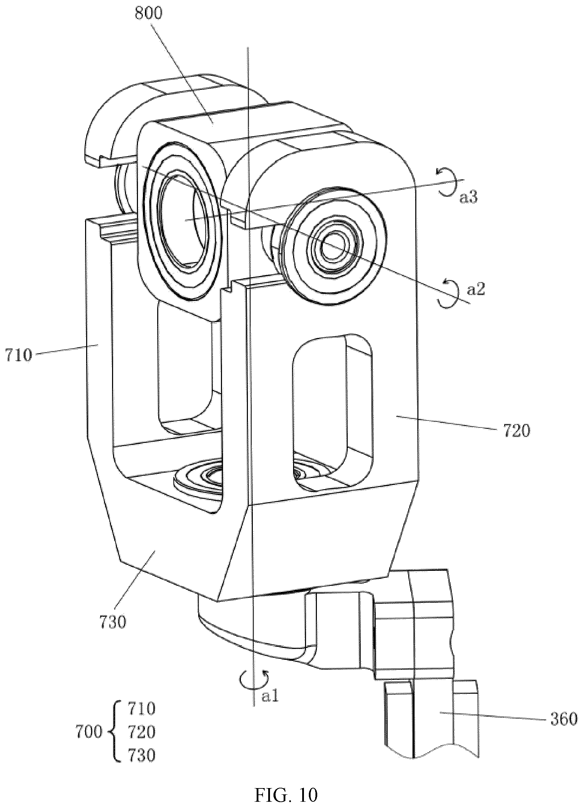

FIGs. 10 to 12 show the schematic structural diagrams of the hinge pair connecting the support member and the movable platform 200 in accordance with the present disclosure.

The hinge pair comprises a first connecting block 700 and a second connecting block 800. The first connecting block 700 is U-shaped and comprises a first support portion 710 and a second support portion 720 opposite to each other, and a connecting support portion 730 connecting one end of the first support portion 710 and the second support portion 720.

The second connecting block 800 is rotatably mounted on the first support portion 710 and the second support portion 720. The connecting support portion 730 can be rotationally connected to the corresponding support member through the first rotating shaft rotating around the first rotating shaft a1. The first shaft hole 731 formed on the connecting support portion 730 can be used to accommodate the first rotating shaft. The second connecting block 800 can be rotationally connected to the movable platform 200 through the second rotating shaft rotating around the second rotating shaft a2. The first rotating shaft a1 is parallel to the extension direction of the first support portion 710 and the second support portion 720, and is perpendicular to the second rotating shaft a2.

The second connecting block 800 is provided with a second shaft hole 830. The central axis of the second shaft hole is perpendicular to the second rotating shaft a2. The output shaft extending from the movable hinge point of the movable platform 200 can be inserted into the second shaft hole 830. Specifically, the output shaft of the movable platform 200 can be installed in the second shaft hole 830 through bearings to enable it to rotate around the central axis of the second shaft hole (i.e., the third rotating shaft a3).

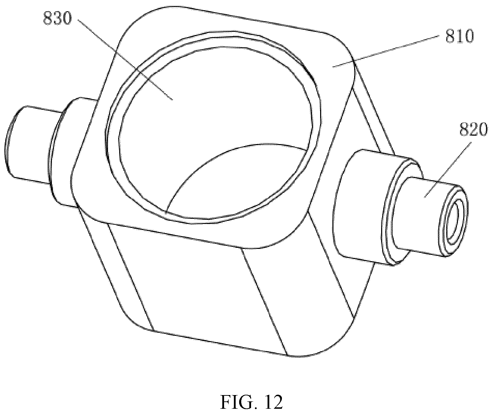

As shown in FIG. 12, the second connecting block 800 may include a main body 810 formed with the above-mentioned second shaft hole 830 and convex shaft 820 protruding respectively from both sides of the main body 810. The two convex shafts 820 can be respectively inserted into the grooves or holes 711 721 formed in the first support portion 710 and the second support portion 720. Specifically, the two convex shafts 820 can be rotatably installed respectively in the grooves or holes 711 721 through bearings to be used as the rotation axes of the second connecting block 800.

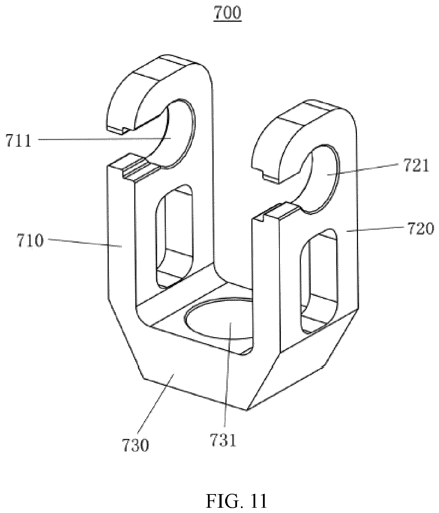

Furthermore, FIG. 11 shows the situation where holes are formed on the first support portion 710 and the second support portion 720. However, this is not limited. Grooves can also be formed on the first support portion 710 and the second support portion 720. Additionally, in order to facilitate the installation of the second connecting block 800 on the first support portion 710 and the second support portion 720, a part of the holes 711 721 can be open.

However, the embodiments of the present disclosure are not limited to this. As long as the hinge pair can achieve rotation around the first rotating shaft al, the second rotating shaft a2, and the third rotating shaft a3, and can restrict the structure where the first rotating shaft a1 and the third rotating shaft a3 coincide, such a structure is feasible.

In the existing technology, the first connecting block 700 is usually L-shaped, that is, the first connecting block 700 only has the first support portion 710 and the connecting support portion 730. In this case, the second connecting block 800 can rotate 360° around the second rotating shaft a2. Since the second connecting block 800 is connected to the movable platform 200, during the rotation of the second connecting block 800 around the second rotating shaft a2, there are two opportunities to align the third rotating shaft a3 with the first rotating shaft a1. The first opportunity occurs when the second connecting block 800 is on the side facing the movable platform 200 with the connecting support portion 730 on the back. The second opportunity occurs when the second connecting block 800 rotates to face the connecting support portion 730 on the side facing the movable platform 200. During the second opportunity, the movable platform 200 will interfere with the L-shaped first connecting block 700, which may cause the movable platform 200 to freeze, limiting the motion range of the movable platform.

According to the embodiments of the present disclosure, by setting the first connecting block 700 in a U-shaped configuration, due to the combined restriction of the first support portion 710 and the second support portion 720, the aforementioned second coincidence can be avoided, thereby preventing the interference between the movable platform and the hinge pair. That is to say, when the second coincidence is about to occur, the first connecting block 700 rotates around the first rotating shaft a1, thereby avoiding the third rotating shaft a3 from coinciding with the first rotating shaft a1 again. Additionally, since the first connecting block has a symmetrical U-shaped structure, there is no restriction on the motion range of the movable platform 200.

In one implementation mode, the master controller can be implemented as a 3-UPS parallel mechanism. That is, each support member can be implemented using the UPS branch chain, enabling the movable platform to have 6 degrees of freedom, thereby allowing the master controller to achieve 6 degrees of freedom of motion and 6 degrees of freedom of force feedback. For example, each support member can be connected to the static platform through the U pair and to the movable platform through the S pair. **In** addition, a mobile P pair can be provided on each branch chain to enable it to elongate or shorten.

According to the embodiments of the present disclosure, by setting the included angle between the static platform and the horizontal plane to be less than 45°, the gravity can be balanced to a large extent, while reducing the requirements for the rotational drive assembly, reducing the volume of the rotational drive assembly, and increasing the design space of the master controller of the console.

According to the embodiments of the present disclosure, the transmission gap is eliminated by the staggered arrangement of the two gears, avoiding the jitter phenomenon caused by delayed meshing.

According to the embodiments of the present disclosure, by setting the sector gear and the appropriate transmission ratio, the rotational inertia is reduced, the lagging sensation is decreased, and the operator's hand feel is improved.

According to the embodiments of the present disclosure, by designing the hinge pair including the U-shaped connecting member, the interference between the movable platform and the hinge pair is avoided, expanding the motion range of the movable platform.

In the description of the present disclosure, the terms "first" and "second" are only used for the purpose of the description and cannot be understood as indicating or implying relative importance or implying the quantity of technical features indicated. Therefore, features defined with "first" and "second" can explicitly or implicitly include one or more of these features. In the description of the present disclosure, unless otherwise stated, "multiple" means two or more.

In the description of the present disclosure, it should be noted that unless otherwise specified and limited, the terms "installation", "connected with", "connection", and "fixation" should be understood broadly. For example, they can be fixed connection, detachable connection, or integrated connection, mechanical connection, electrical connection, or communication connection; they can be directly connected or indirectly connected through an intermediate medium, or can be the internal connection between two components or the interaction relationship between two components. For the skilled person in the art, the specific meanings of the above terms in the present disclosure can be understood in specific circumstances.

The features, structures, or characteristics described in the present disclosure may be combined in one or more embodiments in any suitable manner. In the above description, many specific details are provided to provide a full understanding of the embodiments of the present disclosure. However, the skilled person in the art will be aware that the disclosed technical solution can be practiced without one or more of the specific details described, or other methods, components, materials, etc. may be employed. In other cases, the well-known structures, materials, or operations are not shown or described in detail to avoid blurring various aspects of the present disclosure.

## Claims

1. A console, the console comprising a master controller, the master controller comprises:
static platform;
movable platform, the movable platform is arranged on one side of the static platform;
the handle, the handle connected to the movable platform; and
the connecting arm, both ends of the connecting arm are respectively hinged and connected to the static platform and the movable platform, the connecting arm includes N support members, where N is greater than or equal to 3, the connecting arm comprises at least the first support member, the second support member, and the third support member, the first support member to the Nth support member are respectively hinged at the first static hinge point to the Nth static hinge point to the static platform,
among them, the master controller also includes N first drive assemblies for driving each support member to rotate relative to the static platform,
among them, the included angle between the static platform and the horizontal plane is less than 45°.

2. The console of claim 1, wherein, the included angle between the static platform and the horizontal plane is less than or equal to 10°.

3. The console of claim 2, the included angle is equal to 0° or 5°.

4. The console of claim 1, the circumference of the static platform and/or the movable platform is divided into the opposite first side and second side in the circumferential direction, relative to the second side in the circumferential direction, the N support member are arranged around the static platform and/or the movable platform on the first side in the circumferential direction, and the angular range of the second side in the circumferential direction is greater than or equal to 150° and less than 300°.

5. The console of claim 4, the angular range of the second side in the circumferential direction can be 180°.

6. The console of claim 4, the N support members are evenly arranged in the circumferential direction on the first side in the circumferential direction; and/or the handle is located at the middle position of the second side in the circumferential direction.

7. The console of claim 4, the N first drive assemblies are arranged in the area on the first side in the circumferential direction; and/or the first drive assembly that drives the first support member is arranged within the projection area of the static platform.

8. The console of claim 7, the first support member, the second support member, and the third support member are respectively connected to the static platform through the first static hinge point, the second static hinge point, and the third static hinge point, the first static hinge point, the second static hinge point, and the third static hinge point are located on the first circle,
the first drive assembly corresponding to the first support member and the first drive assembly corresponding to the third support member are arranged on the same diameter of the first circle, and the output shaft of the first drive assembly corresponding to the first support member and the output shaft of the first drive assembly corresponding to the third support member face the same side.

9. The console of claim 1, the first static hinge point to the Nth static hinge point are respectively connected to the virtual center of the static platform to form the first virtual straight line to the Nth virtual straight line,
among them, an included angle is formed between two adjacent virtual straight lines, and the first virtual straight line to the Nth virtual straight line form N included angles,
among them, the included angle formed between the Nth virtual straight line and the first virtual straight line is greater than or equal to 150° and less than 300°.

10. The console of claim 9, the first support member to the Nth support member are respectively hinged to the movable platform at the first movable hinge point to the Nth movable hinge point,
the movable platform is arranged parallel to the static platform,
among them, the projections of the first support member to the Nth support member from the movable platform to the static platform are respectively coincident with the first virtual straight line to the Nth virtual straight line.

11. The console of claim 9, the Nth included angle is equal to 180°; and/or the sum of the N included angles is equal to 360°, and the other included angles except the Nth included angle are equal to each other.

12. The console of claim 1, the handle is located between the first support member and the Nth support member; and/or the N is an odd number, the projection of the handle and the (N + 1) / 2-th support member on the static platform is located on the same straight line.

13. The console of claim 1, the handle comprises a grip portion, the grip portion is arranged at a predetermined angle with the horizontal plane, and/or the grip portion is arranged at the predetermined angle with the static platform.

14. The console of claim 13, the predetermined angle is from 45° to 90°; and/or the included angle between the predetermined angle and the support member and the static platform is close to or equal.

15. The console of claim 1, the handle is arranged between the static platform and the movable platform; and/or the movable platform is arranged above the static platform, and the first drive assembly are arranged at the lower end of the corresponding support member.

16. The console of claim 1, the driving force of the first drive assembly is transmitted to the corresponding support member through the gear set,
the gear set comprises a driving gear connected to the output shaft of the first drive assembly, and a driven gear rotatably arranged on the static platform and meshing with the driving gear, the rotating shaft of the driven gear is connected to the corresponding support member.

17. The console of claim 16, the driving gear comprises a first driving gear and a second driving gear which are coaxially and side by side arranged and have the same structure, and the driving teeth of the first driving gear and the driving teeth of the second driving gear are arranged out of phase in the rotation direction; and/or the driven gear comprises a first driven gear and a second driven gear which are coaxially and side by side arranged and have the same structure, the driving teeth of the first driven gear and the driving teeth of the second driven gear are arranged out of phase in the rotational direction.

18. The console of claim 17, the offset distance between the driving teeth of the first driving gear and the driving teeth of the second driving gear is approximately equal to the transmission clearance between the first driving gear and the driven gear; and/or the offset distance between the driving teeth of the first driven gear and the driving teeth of the second driven gear is approximately equal to the transmission clearance between the driving gear and the first driven gear.

19. The console of claim 16, the driven gear is a sector gear, the sector angle of the sector gear is from 60° to 150°, and the sector gear is set to hollowed-out shapes; and/or the transmission ratio between the driving gear and the driven gear is from 2:1 to 10:1.

20. The console of claim 1, each of the first support member, the second support member, and the third support member comprises a connecting bracket hinged to the static platform, a drive rod moving along the connecting bracket, and a second drive assembly arranged on the connecting bracket, the second drive assembly comprises a mover and a stator, and the mover is fixedly connected to the drive rod and moves synchronously; and/or each support member is rotationally connected to the movable platform through a hinge pair, and the hinge pair comprises:
the first connecting block, the first connecting block is U-shaped, comprises the first support portion and the second support portion opposite to each other, and the connecting support portion connecting one end of the first support portion and the second support portion; and
the second connecting block is rotatably mounted on the first support portion and the second support portion,
among them, the connecting support portion is rotatably connected to the corresponding support member through the first rotating shaft, and the second connecting block is rotatably connected to the movable platform through the second rotating shaft,
among them, the central axis of the first rotating shaft is parallel to the extension direction of the first support portion and the second support portion, and is perpendicular to the rotating shaft of the second connecting block rotating on the first support portion and the second support portion.

21. The console of claim 9, the console comprising two master controllers, the two master controllers are symmetrically arranged, and the openings of the Nth included angle on both master controllers are oriented towards the same side,
among them, the projection of the handle on the static platform is located within the Nth included angle.

22. The console of claim 1, the console comprising two master controllers, and the two master controllers are symmetrically arranged on the bottom plate of the console,
among them, the four first drive assembly that respectively drive the first support member and the third support member of the two master controllers to rotate around their respective static hinge points are arranged along the same straight line.

23. The console of claim 22, the first drive assembly that respectively drives the second support member of the two master controllers to rotate around their respective static hinge points, is arranged in parallel; and/or the two first drive assembly that respectively drive the first support member of the two master controllers are located beneath the static platform.

24. A robot, the robot comprising a console according to any one of claims 1 to 23.

25. The robot of claim 24, the robot is a surgical robot.
